# EUROPEAN PATENT APPLICATION

(11) **EP 1 810 612 A1**
(43) Date of publication of application: **25.07.2007**
(21) Application number: 06100653.2
(22) Date of filing: 20.01.2006
(51) Int. Cl.: A61B 5/022, G06F 19/00

(54) **A system and method for hypertension management**

(71) Applicant: Microlife Intellectual Property GmbH, 9443 Widnau (CH)
(72) Inventor: Kin Yuan Lin, Taipei, 114 (TW)
(74) Representative: Müller, Christoph Emanuel

(57) **Abstract**

A system (1) for hypertension management comprises at least one home blood pressure measuring device (10) for blood pressure measurements of an individual at the individual's home. Furthermore, the system (1) comprises at least one point of care measuring system (20) with a point of care blood pressure measuring device (21). This allows blood pressure measurements at a point of care. The system is provided with data communication means for transferring data from the home blood pressure measuring device (10) to a web server (35) or the point of care measuring system (20).

## Description

The invention relates to a system and a method for hypertension management. Hypertension is a major cause for severe diseases in most industrialised countries, and it is growing significantly in developing and poor countries. In view of treatment of hypertension it is thus important to provide reliable blood measurement results of individuals. In traditional patient care processes, patients, physicians and pharmacists are different, isolated entities. Patient's data or medical records are not circulated or shared among these parties. More importantly, methods and procedures for patients to take blood measurements at home and also methods for point of care measurements are not standardised. Consequently, information needed for diagnosis, treatment or medication adjustment for hypertension may be inaccurate. There have been no incentives to physicians or pharmacists to use standardised guidelines for diagnosis, treatment or for providing sufficient consultations and education materials or services. The overall control rate for hypertension management therefore is only 21% - 27% (see e.g. Judy Possidente Kaufman, etc., "The role of Home Blood Pressure Monitoring in Hypertension Control", Journal of Clinical Hypertension 3(3): 171-173, 2001). Current hypertension management systems thus are not successful.

There are current proposals to start pay for performance programs (P4P) for physicians in view of reimbursements regarding treatment of hypertension. Similarly, medication therapy management (MTM) programs are currently planned in view of reimbursements for pharmacists in order to make sure drug compliance. In these programs, specific documentation is required as evidence to prove that physicians and pharmacists have provided necessary services, follow-up and keeping track of hypertension treatment outcomes.

It is an object to overcome the drawbacks of what is known, in particular to provide a system and a method for hypertension management which allows achieving a high overall control rate. Furthermore, the system and method according to the present invention shall provide reliable blood pressure measurements upon which a treatment of an individual can be based. It is a further object of the invention to provide a method and a system for hypertension management which easily allows doctors or pharmacists to comply with requirements of quality standard programs such as P4P or MTM programs.

According to the present invention, these and other objects are solved with a system and a method according to the independent patent claims.

The system according to the invention is primarily used for hypertension management. The system includes at least one home blood pressure measuring device for blood pressure measurement at an individual's home. Of course, depending on the number of individuals treated with such a system, there can be a plurality of such home blood pressure measuring devices. The system further comprises at least one point of care measuring system. The point of care measuring system includes a point of care blood pressure measuring device for blood pressure measurement of an individual at a point of care. Typically, a point of care in this context is a doctor's office, a pharmacy or any other kind of centralised health care institution. According to the invention, the system is provided with data communication means for transferring data from the home blood pressure measuring device to a central web database accessible by a care person such as a doctor or a pharmacist or for transferring data to the point of care measuring system. The system allows to combine blood pressure measurements made at the individual's home and measurements done by a doctor or a pharmacist at a point of care. While it is known that home measurements may be the best alternative for accurate blood pressure measurements (see e.g. Laurie Barclay, "Home measurement best for accurate BP monitoring", BMJ.2002; 325:254-257 or Judy Possidente Kaufman et al., "The role of home blood pressure monitoring in hypertension control", J Clin Hypertens 3(3): 171-173, 2001), it is generally accepted that physicians or pharmacists should in addition make their own blood pressure measurements for verification purposes. According to the system of the present invention, a patient may take a plurality of home blood pressure measurements. Data of these measurements can be made accessible to care persons, doctors, physicians by uploading the data to a database, in particular a web based database or by uploading the data to the point of care measurement system through the data communication means. Direct uploading of the data at the point of care will be particularly preferable for patients or individuals which are not used to utilize computers.
According to a preferred embodiment of the invention, the home blood pressure measuring device is adapted to determine blood pressure in accordance with specific, predetermined measurement criteria. Such measurement criteria based on clinical considerations are known. A device for carrying out such measurements is disclosed in the co-pending PCT application PCT/EP2005/052730, the content of which hereby is incorporated by reference.

According to still a further embodiment of the invention, the point of care blood pressure measuring device is adapted to determine blood pressure in accordance with specific, predefined measurement guidelines. Such guidelines are e.g. known from Pickering et al. (Pickering et al., Recommendations for blood pressure measurements in humans and experimental animals, Hypertension 2005; 45: 142-161). A point of care blood pressure measuring device appropriate for such measurements is disclosed in the co-pending application having same filing date in the name of same applicant which is incorporated herein by reference.

According to another aspect of the invention, the system includes at least one point of care measuring device for blood pressure measurements at a point of care. The point of care measuring system is designed to operate in accordance with predetermined quality standards, in particular in accordance with the criteria of a pay for performance program or in accordance with the criteria of a medication therapy management program. Such a device easily allows a doctor to generate reports necessary to fulfil the requirements of such a program. In particular, the point of care blood pressure measuring device may generate reports necessary for the physician or pharmacist to be reimbursed for his or her services. While such a device with a capability to generate quality standard reports already has considerable advantages as such, it will be understood that in combination with the above mentioned integrated system having home and point of care measuring devices, additional advantages can be achieved.

According to a further aspect of the invention, the system may further comprise at least one home based medication compliance monitoring device. This device may be designed for data communication with the central database. In addition to transmission of blood pressure data, medication compliance data of the patient may be transmitted therewith. In particular, if a point of care system is arranged at the doctor's and at the pharmacist's premises, medication compliance and blood pressure data which are relevant for pharmacists or physicians can be accessed to the point of care system.

In another preferred embodiment, the system is provided with a central database. This database particularly may be a web based database. In the database, blood pressure data and drug compliance data may be stored. Thereby, these data are in parallel accessible by a plurality of users such as a doctor, a pharmacist, the patient, nurses or other care givers. Knowledge of physicians and pharmacists and measurements done by patients thereby can be integrated. With the system of the present invention, patients, physicians and pharmacists and their measurements are not isolated anymore. They can be integrated in one overall system. General information about disease management knowledge can be downloaded on demand if required by a physician or a pharmacist.

Based on such a database it is further possible to generate individual education reports for each patient. Depending on the patient's condition, in particular depending on blood pressure values but also parameters such as age, weight or the like, individual reports may be generated.

In view of the above, it is particularly preferred if according to the system of the present invention there is provided a point of care system at least one doctor's office and at least one pharmacy.

Another aspect of the invention relates to a blood pressure measuring device for blood pressure measurements at a point of care. The automatic blood pressure measurement device is provided with means for generating reports to be used in a quality control program, in particular in a pay for performance or in a medication therapy management program. The blood pressure measuring device typically is a standard blood pressure measuring device for automatic blood pressure measurements. In addition, this device is provided with software instructions, which allow the generation of reports fulfilling requirements of such programs. In particular, a report in the form of a prospective data collection flow sheet as suggested in "Clinical performance measures, Hypertension, Tools developed by physicians for physicians" provided by the American College of Cardiology/American Heart Association/Physician Consortium for Performance Improvement can be automatically generated with the device according to the invention. The reports typically may include, but no limited to, all data records, clinical performance measures, plan of care, etc.

According to a further aspect of the invention, there is provided a method for hypertension management. According to this method, in a first step, blood pressure values of an individual are determined at the individual's home with a home blood pressure measuring device. In a further step, blood pressure values of the individual are determined at a point of care with a point of care blood pressure measuring device. The blood pressure data measured at the individual's home are then transferred from the home blood pressure measuring device to a central database or to a point of care blood pressure measuring system.

It is preferred that blood pressure data determined at the individual's home and/or blood pressure data determined at the point of care are determined in accordance with respective recommendation or measurement criteria.

According to a further aspect of the invention, in a method for hypertension management, in a first step blood pressure values of an individual are determined at a point of care. In a further step, reports used in a quality insurance program such as a pay for performance or a medication therapy management program are automatically generated by the software tool.

According to a further preferred embodiment of the invention, in addition to the blood pressure data, medication compliance data determined in a medication compliance monitoring device at the individual's home are also transferred to a central database or to a point of care blood pressure measuring system.

According to a further preferred embodiment of the method according to the invention, data relating to the patient, further individual data such as blood glucose, weight, age or the like are transferred to the central database, in particular an internet database, which can be accessed from several point of care systems. Furthermore, individual education reports for an individual can be automatically generated from such a system.

The invention will now be explained in more detail with reference to the following embodiments and the accompanying drawings, in which
- Figure 1: shows a schematic representation of components of a system according to the present invention
- Figure 2: shows a flow chart with steps of a method according to the present invention
- Figure 3: shows a general overview of a system according to the invention
- Figure 4: shows a block diagram of different components and mutual communication of a system according to the invention and
- Figure 5: shows an example of a P4P report.

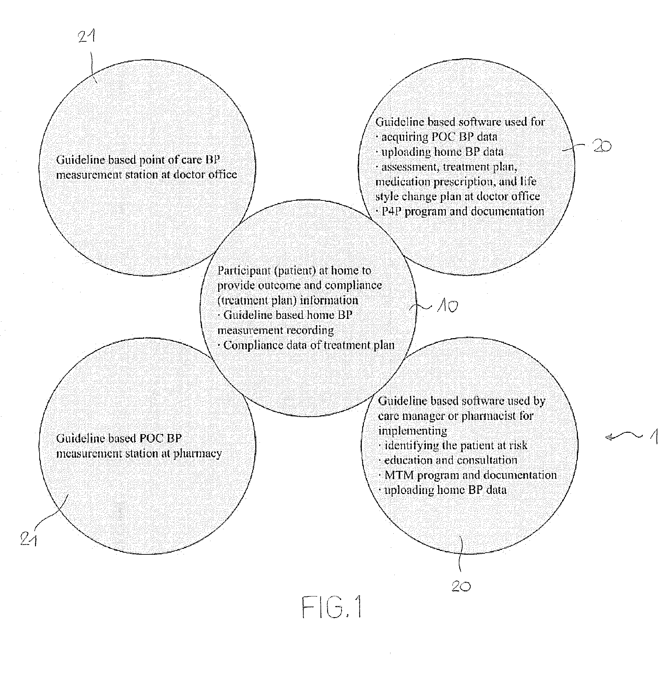

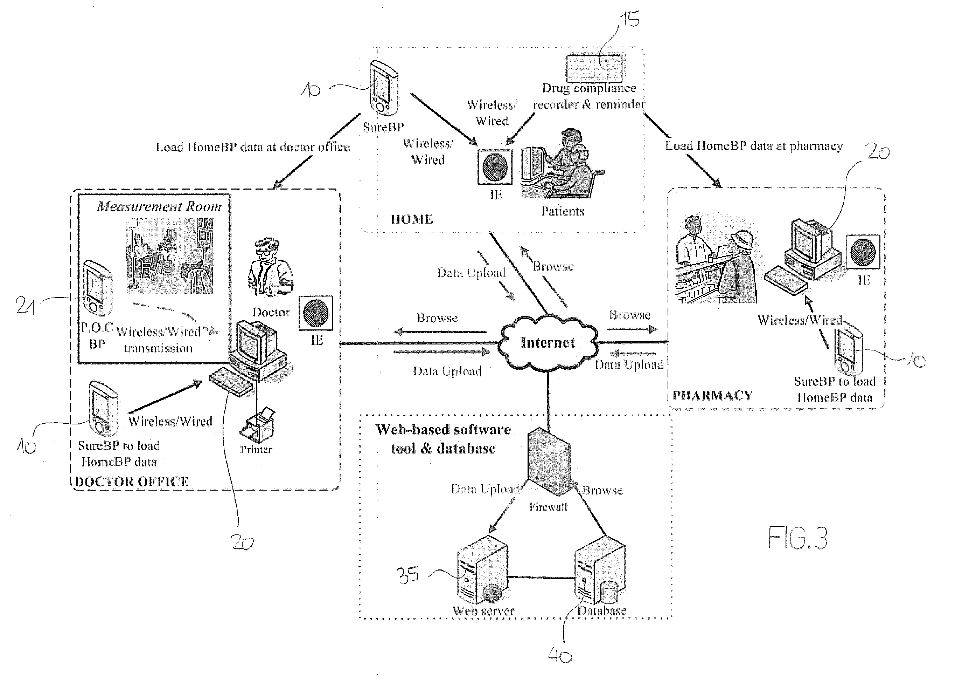

Figure 1 schematically shows several components of a system 1 for hypertension management in accordance with the present invention. A first component includes a home blood pressure measuring device 10. An individual or patient is carrying out blood pressure measurements with the home blood pressure measuring device 10 in accordance to specific guidelines. In addition, compliance data for treatment plan are recorded by a drug compliance recorder device at the patient's home (see Fig. 3)

A first point of care measuring system 20 is located at a physician's office. The point of care measuring system 20 includes guideline based software used for several purposes. The software could also be on a web server for physicians to be used online, e.g. by logging in through the internet (see Fig. 3). The software is used for acquiring blood pressure data at the point of care according to specific guidelines. The software is furthermore used to upload data which have been measured by the individual at the individual's home with the home blood pressure measuring device 10. Included in the software is furthermore information relating to prescription, assessment, treatment plans or lifestyle change plans. Finally, the software used in the system is capable of generating reports and documentation in response to a pay for performance program.

A second point of care measuring system 20 is used at a pharmacy. The system includes guideline based software used by a care manager or a pharmacist for implementing different tasks.

The software could be locally at pharmacist's computer or on the web server 35 for pharmacists for online use. One of the task is to identify whether a specific individual or patient is at risk. Another task of the software is to provide education and consultation information. Furthermore, the software is adapted to provide reports in answer to requirements of an MTM program and appropriate documentation. Finally, the software is also used for uploading data from a home blood pressure measuring device 10 which data have been acquired individually by the patient. With a point of care blood pressure measuring device 21 point of care blood pressure measurements may be made at a doctor's office or at a pharmacy.

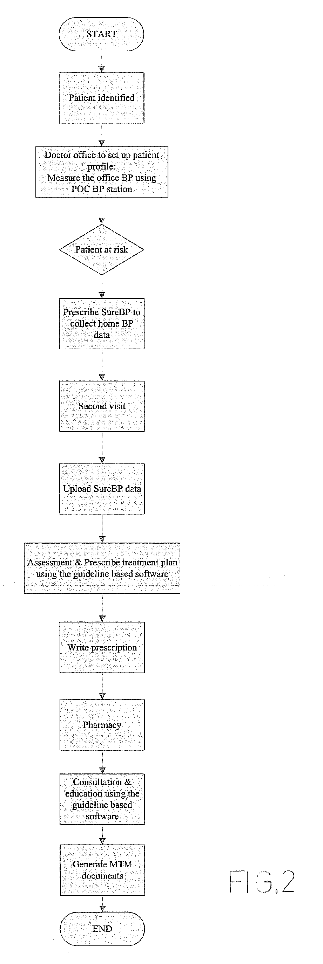

Figure 2 shows a flow chart of operation of the method according the invention carried out at a doctor's office. In a first step after start of the procedure a patient is identified. In the doctor's office, a patient profile is set up. With the point of care blood pressure measurement device 21 the individual's blood pressure is measured. If the patient is not at risk, the method according to the invention is stopped. If the patient is found to be at risk to suffer from hypertension, he is prescribed with a home blood pressure measuring device 10 (called SureBP herein after) to collect home blood pressure data. The home blood pressure measuring SureBP 10 is operating in accordance with predefined measurement criteria. In particular, the home blood pressure measuring device 10 is programmed so as to automatically remind the patient to take measurements at appropriate times.

In a second visit, the patient or individual uploads the blood pressure data measured at home with the home blood pressure measuring device 10. This may be e.g. done by connecting the home blood pressure measuring device 10 through an interface to the point of care measuring system 20. Upload of the data can also be made online e.g. by uploading the data to an internet database.

In a further step, an assessment of the patient is made and an appropriate treatment plan is prescribed. For prescription of the treatment plan, a guideline based software is used. Typically, the software includes guidelines such as recommended by WHO/ESH/NIH. Based on the assessment and the treatment plan, a prescription is written and the individual is referred to a pharmacy. At the pharmacy, consultation and education of the individual is made on the basis of a guideline based software running at the pharmacy. Furthermore, the point of care system 20 at the pharmacy generates MTM documents necessary for the pharmacist to be reimbursed.

The communication interface for uploading data from the home blood pressure measuring device to the point of care measuring device e.g. may be a USB connection. Any other kind of connection may be conceivable.

By transferring data to an internet based database, trends, medication compliance or other information may be presented to the physician, pharmacist or to other care givers.

Medication compliance may be made with a programmed medication schedule device. On a screen, reminders for taking medication may be provided. Confirmation of compliance may be entered into the device. The medication schedule can be synchronised online.

Figure 3 shows a general overview of the system according to the present invention. Mutual communication between the different components are shown. At the patient's home, data are transferred from a drug compliance recorder 15 and reminder and/or from a home blood pressure measuring device 10 to a web database 40 through the internet. Through the internet, contents of this database also may be browsed. Blood pressure data also are stored in the home blood pressure measuring device 10, so that they can be transmitted to a point of care measuring system 20 if the device 10 is brought to the point of care. Data can be uploaded from this point of care measuring system 20 to the central database 40 through the internet so that they will be accessible for all the parties involved. Software may be made available on a web server 35.

A point of care system 20 is arranged each at a doctor's office and at a pharmacy. Data gathered at these locations, in particular data measured by a point of care blood pressure measuring device 21 or prescription or consultation data may be uploaded to the database 40 through the internet. Furthermore, data contained in the database 40 can be accessed from the pharmacy or from the doctor's office through the internet. In particular, relevant measurement data but also knowledge information can be accessed.

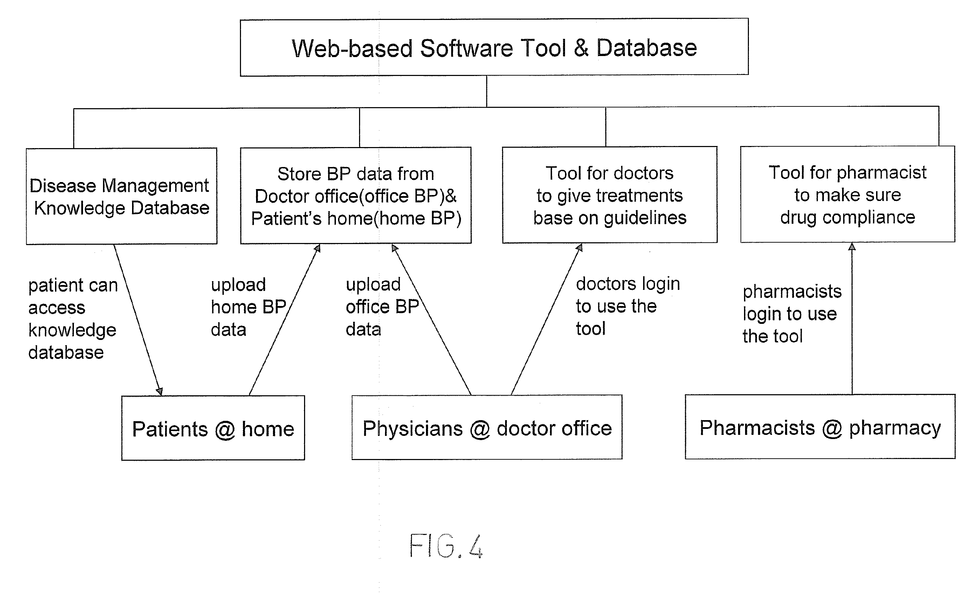

Figure 4 shows in more detail contents of the web-based software tools and database and interactions thereof with different users. In particular, a patient can access a disease management knowledge database from home and the patient can upload home blood pressure data measured with the home blood pressure measuring device 10 to the central web database where they are stored. The physician at the doctor's office can also upload blood pressure data which have been gathered with the point of care measuring device 21 to the database 40. In addition, a physician can access specific tools in view of treatments based on guidelines. Pharmacists can access to tools in order to ensure drug compliance. Furthermore, patients can upload compliance data to the web database 40.

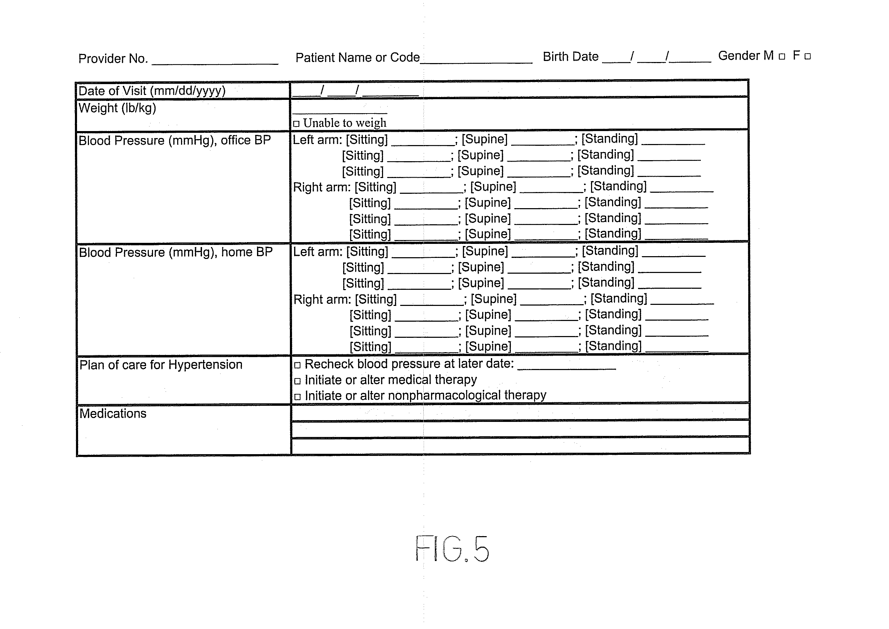

Figure 5 shows an embodiment of a P4P report which will be completed by means of the present invention.

## Claims

1. A system (1) for hypertension management, the system comprising
- at least one home blood pressure measurement device (10) for blood pressure measurements at an individual's home
- at least one point of care measuring system (20) with a point of care blood pressure measuring device (21) for blood pressure measurement at a point of care,
wherein the system (1) is provided with data communication means for transferring data from said home blood pressure measuring device (10) to a central data base (40) and/or said point of care measuring system (20).

2. A system (1) according to claim 1, wherein said home blood pressure measuring device (10) is adapted to determine blood pressure values in accordance with predetermined measurement guidelines.

3. A system (1) according to one of the claims 1 or 2, wherein said point of care blood pressure measuring device (21) is adapted to determine blood pressure values in accordance with predetermined measurement guidelines.

4. A system, in particular in accordance with one of the claims 1 to 3, comprising at least one point of care measuring device (21) for blood pressure measurements at a point of care,
wherein said point of care blood pressure measuring device is designed to operate in accordance with predetermined quality standards, in particular in accordance with guidelines of a pay for performance (P4P) program or in accordance with a medication therapy management program (MTM).

5. A system according to one of the claims 1 to 4, the system further comprising at least one home based medication compliance monitoring device, said medication compliance monitoring device being designed for data communication with central database.

6. A system according to one of the claims 1 to 5, wherein the system (1) is further provided with a centralised web based software tool and database (40) accessible by at least one of the point of care measuring system (20) and the home blood pressure measuring system.

7. A system according to claim 6, wherein the web based database includes a disease management knowledge database accessible by the individual.

8. A system according to one of the claims 6 or 7, wherein the web based database includes tools for doctors in view of providing treatments based on guidelines.

9. A system according to one of the claims 6 to 8, wherein the web based database includes tools for pharmacists to ensure drug compliance.

10. A system in accordance with claim 4 and one of the claims 6 to 9, comprising web based software tools in view of generation of reports in accordance with predetermined quality standards.

11. A system according to one of the claims 1 to 10, wherein the system includes at least one point of care measuring system (20) at a doctor's office and at least one point of care measuring system at a pharmacy.

12. A system according to one of the claims 1 to 11, the system further comprising means for generating individual education reports for said individual.

13. A blood pressure measuring device for blood pressure measurements of an individual at a point of care, wherein the blood pressure measurement device (21) is provided with means for generating reports to be used in a quality insurance program, in particular in a pay for performance or a medication therapy management program.

14. A method for hypertension management, the method comprising the steps of
- determining blood pressure values of an individual (I) at the individual's home with a home blood pressure measuring device (10)
- determining blood pressure values of said individual at a point of care with a point of care blood pressure measuring device (21)
- transferring data from said home blood pressure measuring device (10) to a point of care blood pressure measuring system (20) and/or a central database (40).

15. A method according to claim 14, wherein determination of blood pressure values of the individual at the individual's home is made in accordance with predetermined measurement criteria.

16. A method according to one of the claims 14 or 15, wherein determination of blood pressure values at the point of care is made in accordance with predetermined measurement criteria.

17. A method for hypertension management, in particular in accordance with one of the claims 14 to 16, comprising the steps of
- determining blood pressure values of an individual (I) at a point of care with a point of care blood pressure measuring device (21)
- automatically generating reports for a quality standard program, in particular reports for a pay for performance program or for a medication therapy management program.

18. A method according to one of the claims 14 to 17, comprising the further step of gathering home based medication compliance information with a home based medication compliance monitoring device and transmitting said compliance data to a central database (40).

19. A method according to one of the claims 14 to 18, comprising the further steps of generating education reports for said individual based on information contained in a data base of said management system (1).

20. A method according to one of the claims 14 to 19, wherein the individual accesses a disease management knowledge database arranged on a central web database (40).

21. A method according to one of the claims 14 to 20, wherein blood pressure data determined by the individual at home with a home blood pressure measurement device (10) and blood pressure data determined at the point of care with the point of care blood pressure measuring device (21) are transmitted to a central database (40), preferably a web based database.
